# EUROPEAN PATENT APPLICATION

(11) **EP 4 295 921 A2**
(43) Date of publication of application: **27.12.2023**
(21) Application number: 23179778.8
(22) Date of filing: 16.06.2023
(51) Int. Cl.: A62B 18/00, A61F 9/06, B23K 9/32

(54) **BLOWER FOR POWERED PAPRS AND WELDING MACHINE FOR POWERING PAPRS**

(30) Priority: 22.06.2022 GB 202209134
(71) Applicant: World Wide Welding Limited, Lutterworth LE17 4AT (GB)
(72) Inventor: Beddoes, Gareth, Wolverhampton WV9 5HF (GB); Jordan, James, Wolverhampton WV9 5HF (GB); Mackenzie-Grist, Alexander, Wolverhampton WV9 5HF (GB)
(74) Representative: Meissner Bolte Partnerschaft mbB

(57) **Abstract**

The present disclosure relates to a blower (400) for a powered air purifying respirator (PAPR), the blower (400) comprising a modular housing (402) including: a first configuration, in which the housing (402) comprises a removable battery (420) for supplying the blower with power, and a second configuration, in which the housing (402) comprises a removable power converter (430) for supplying the blower with power, the removable power converter being connectable to a mains power outlet, wherein the modular housing has substantially identical contours in its first and second configuration.

## Description

### Background of the Disclosure

The disclosure relates to a blower, particularly a blower for a powered air purifying respirator and a welding machine for powering a welding tool. Other aspects of the present disclosure relate to a welding kit.

It is generally known to utilise powered air purifying respirators (PAPRs) in industrial applications with environmental hazards. Such environmental hazards may include respiratory hazards such as gases, vapours, and/or particulate matter that is harmful to the respiratory system of a human being. One such hazardous environment is a welding site. During welding, a welder risks being exposed to hazardous particulates that could lead to various forms of illness and disease, such as cancer and respiratory-related issues.

PAPRs provide purified (e.g. filtered) air to the user when operating in the above environments. To this end, PAPRs usually include a blower equipped with a fan/impeller for generating a forced air flow. The blower typically includes a blower housing with an inlet port and an outlet port. In some examples, the inlet port is connectable to a removable air filter. Air flow that is created by the blower is sucked into the blower housing via said air filter and provided to a face mask worn by the user via the aforementioned outlet port. Air flow exiting the blower housing via the outlet port is typically provided to a face mask (or respirator) worn by the user via a hose.

Typically, PAPRs are portable systems, in which the blower can be carried around by the user, e.g. via a belt or strap connected to the blower housing. To enable maximum freedom of movement, the blower housings also typically include power dense battery packs, such as lithium ion batteries that are rechargeable and arranged within the blower housing. Much like electrical power tools, the use of PAPRs that are battery-driven requires the operator to regularly recharge the batteries. This process is not only cumbersome but can also lead to downtime between users if the batteries are not charged when the blower is not in use. It is also known that batteries deteriorate over time and require expensive replacements. This is catalysed by the ever-increasing lithium demand, due to the introduction of electric vehicles, driving the purchase price for batteries.

In view of the above, there is a need for alternative ways of powering a PAPR blower that is longer-lasting and more convenient to use.

### Summary of the Disclosure

Aspects and embodiments of the disclosure provide a welding machine and a blower.

In a first aspect of the present disclosure, there is provided a blower for a powered air purifying respirator (PAPR), the blower comprising a modular housing including:
- a first configuration, in which the housing comprises a removable battery for supplying the blower with power, and
- a second configuration, in which the housing comprises a removable power converter for supplying the blower with power, the removable power converter being connectable to a mains power outlet,
wherein the modular housing has substantially identical contours in its first and second configuration.

In one embodiment, the removable power converter comprises a transformer and/or an AC/DC converter.

In another embodiment, the removable power converter is connectable to a mains power outlet via a welding machine to receive a power input.

According to another aspect of the present disclosure, there is provided a welding machine for providing power to a welding tool, wherein the welding machine is configured to power a blower of a powered air purifying respirator (PAPR). The ability of the welding machine to power a PAPR blower advantageously eliminates the need for the blower to include a battery. The blower is therefore free from problems caused by batteries such as charging or requiring expensive replacement after a certain number of uses. The welding machine of the present invention thus also functions as an integrated power supply solution, effectively being able to power all of the equipment required by the welder.

In another embodiment, the welding machine may be connectable to a mains power supply. The welding machine being connectable to a mains power supply advantageously provides a continuous and reliable power supply to the blower.

In another embodiment, the welding machine may be configured to receive an input power and provide a first power output for powering the blower and a second power output for powering a welding process. The first power output may be different from the second power output.

In another embodiment, the welding machine comprises a converter to convert mains power into the first and second power output.

In another embodiment, the welding machine comprises an AC/DC converter configured to convert AC mains power into a DC first power output.

In another embodiment, the welding machine is configured to vary a speed of an electric motor of the blower.

In another embodiment, the welding machine is configured to vary the first power output to vary the speed of the electric motor.

In another embodiment, the welding machine comprises a controller, wherein the controller is configured to:
- receive blower-data representative of one or more blower characteristics;
- determine, on the basis of the blower-data, a suitable input power for the blower; and
- adjust a second power output of the welding machine for powering the blower to said input power.

In another embodiment, the blower-data comprises one or more of:
- blower manufacturer details;
- blower model details;
- a current impeller speed;
- a current air flow rate provided by the blower.

In another embodiment, the welding machine comprises a controller, wherein the controller is configured to:
- receive welding-data representative of current welding parameters of the welding machine;
- determine, on the basis of the welding-data, a suitable air flow rate of purified air to a respirator; and
- provide a control-signal for adjusting a second power output provided to the blower.

In another embodiment, the welding-data comprises one or more of:
- a welding current;
- a welding voltage;
- a feed rate.

According to another aspect of the present disclosure, there is provided a welding kit comprising:
- one of the welding machines described above; and
- a blower for providing purified air to a respirator.

In one embodiment, the welding machine and the blower are connected or connectable via a single cable configured to transfer power and data.

In another embodiment, the blower comprises an impeller for generating forced clean air flow, the electric motor being configured to drive the impeller.

In another embodiment, the blower comprises a battery configured to be recharged when the blower is powered by the welding machine.

In one embodiment, the welding kit comprises a respirator mask connected or connectable to the blower via an air supply duct.

Within the scope of this application it is expressly intended that the various aspects, embodiments, examples and alternatives set out in the preceding paragraphs, and the claims and/or the following description and drawings, and in particular the individual features thereof, may be taken independently or in any combination. That is, all embodiments and all features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

### Brief Description of the Drawings

There now follows a description of preferred embodiments of the disclosure, by way of non-limiting example, with reference being made to the accompanying drawings in which:
Figure 1 is a schematic representation of a powered air purifying respirator;
Figure 2 is a schematic, perspective view of a welding kit according to an embodiment of the present disclosures;
Figure 3 is a schematic diagram of functional parts of a welding machine according to an embodiment of the present disclosure;
Figures 4A to 4C are schematic diagrams of a blower according to an embodiment of the present disclosure.

### Detailed Description of the Drawings

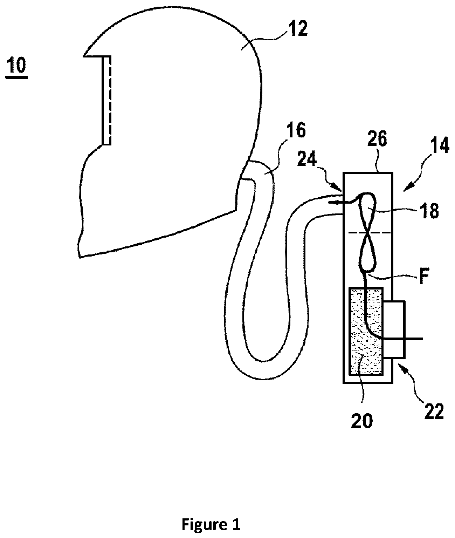

Figure 1 shows a powered air purifying respirator (PAPR). The PAPR 10 shown in Figure 1 is an example of a PAPR that is used in welding applications. To this end, the PAPR 10 of Figure 1 comprises a face cover 12 that is a welding helmet.

However, it should be appreciated that the face cover of the PAPR of the present disclosure could equivalently be any other tight or loose fitting face cover/mask, such as a half mask.

The face cover 12 provides a cover for a user's face and mouth while the user is performing their duties. The face cover protects at least mouth and nose of the user from an external atmosphere and defines a breathing zone. The face cover 12 includes an inlet port through which air may be introduced into the breathing zone of the face cover 12, e.g. via a hose 16.

The face cover 12 is connected to a blower 14 via a hose 16. A first end of the hose 16 is connected to an air outlet port 24 of the blower 14. A second, opposite end of the hose 16 is connected to the air inlet port of the face cover 12. In other words, the hose 16 transfers clean air flow from the blower 14 into the breathing zone of the face cover 12.

The blower comprises a blower housing 26 with an air inlet port 22. A fan or impeller 18 is received within the housing 26 of the blower 14. The impeller 18 is arranged to provide a forced air flow to the breathing zone inside the face cover 12. When in operation, the impeller 18 sucks air into the housing 26 via the air inlet port 22 and transfers the air towards the face cover 12 via the air outlet port 24. In order to purify the air introduced into the blower housing 26, the blower 14 further comprises a filter element 20 arranged within or attached to the outside of (not shown) the blower housing 26. Air sucked into the air inlet port 22 of the blower housing 26 is, therefore, forced through the filter element 20, via flow path F. The air flow is then directed into the hose 16 and face cover 12 via the outlet port 24.

Although this is not specifically shown in Figure 1, the impeller 18 is driven by an electric motor. The electric motor is included in the blower housing 26. In order to power the electric motor (not shown), PAPR systems typically include large lithium ion batteries arranged within the housing. These batteries have a limited lifetime and require recharging on a regular basis. What is more, the large batteries significantly increase the weight of the blower 14, which is typically strapped to the user's body, e.g. via a belt connected to the blower housing 26. The present disclosure provides a solution that does not require large batteries for powering the blower. In some embodiments, the blower may not include a battery at all. In other embodiments, the blower may include a significantly smaller battery, simply as a safety measure.

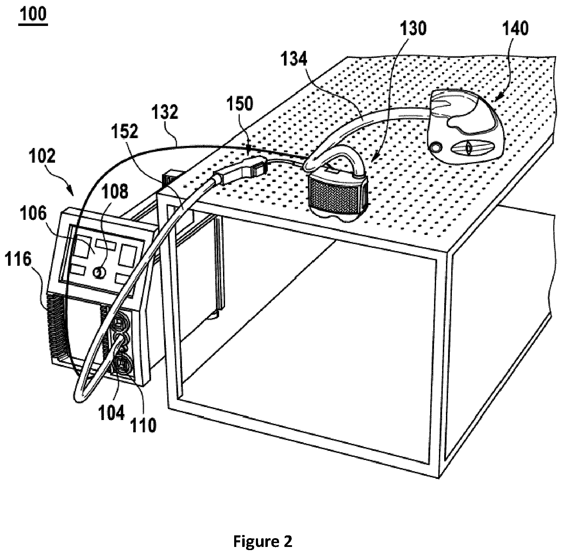

Turning to Figure 2, there is shown a welding kit 100. The welding kit comprises a welding machine 102. The welding machine 102 is configured to provide power to a welding tool 150. In the embodiment shown in Figure 2, the welding machine is an arc welding power supply that provides electric current to the welding tool 150 (e.g. welding electrode). Depending on the welding process used, the welding machine 102 may be configured to provide an alternating current (AC) or a direct current (DC) to the welding tool 150.

In order to supply power to the welding tool 150, the welding machine 102 comprises a first power outlet 104. The first power outlet 104 is connected to the welding tool 150 via a weld cable 152. The welding machine 102 is connectable to a mains power supply. In order to supply the correct voltage and current to the welding tool 150, the welding machine 102 may comprise one or more converters, as is known in the art. These converters may include a combination of transformers, AC/DC converters, and smoothing filters.

The welding machine 102 of Figure 2 comprises a display panel 106 for providing the operator with various welding parameters, such as the welding voltage and welding current currently supplied to the welding tool 150. A rotary dial 108 is shown representative of one or more selectors of the welding machine 102, which may be used by the operator to change the current and voltage supplied to the welding tool, as required. The changes in current and voltage may again be displayed via the display panel 106 of the welding machine 102.

In some examples, the first power outlet 104 for the welding tool 150 may provide electrical power between 15V and 45V at a current of 50A to 600A.

The welding machine 102 further comprises a second power outlet 110. The second power outlet 110 of the welding machine 102 is configured to provide power to a blower 130. The second power outlet 110 may be connected to the blower 130 via a blower cable 132. The welding machine 102 is configured such that a second power output provided via the second power outlet 110 is suitable for driving the blower 130. In other words, the voltage and current provided by the welding machine 102 via the second power outlet 110 may be suitable to drive the electric motor (not shown) of the blower 130. In one example, a second power output provided by the second power outlet 110 may be in the region of 2-10 volts DC at a current of 0.5Ato 5A.

The blower 130 is connected to a face mask 140, e.g. a welding mask, via an air supply hose 134.

As will be described in more detail below, the blower cable 132 may not be exclusively used for power transfer between the welding machine 102 and the blower 130. Rather, the blower cable 132 may also be used to transfer data between the welding machine and the blower 130. In one example, the second power outlet 110 may be a USB-C port and the blower cable 132 may be a USB-C cable, enabling power and data transfer at the same time.

The welding machine 102 also comprises one or more air vents 116 for cooling the welding machine 102. In some embodiments, the welding machine 102 may also comprise a fan for forced air cooling of the welding machine 102.

In some embodiments, the blower 130 shown in Figure 2 may comprise a battery that is configured to be recharged when the blower 130 is powered by the welding unit 102. The blower 130 of this embodiment only comprises a comparatively small battery for continued use of the blower 130 when the blower 130 is no longer connected to the welding machine 102 or if the welding machine 102 should fail to deliver the required power to drive the blower.

In some embodiments, the battery included in the blower 130 of this embodiment can be considered as a safety feature to maintain operation of the blower for a short period of time in case power is no longer provided by the welding machine. The battery, therefore, provides the operator with sufficient time to leave the hazardous environment before the blower's clean air supply is interrupted. In such embodiments, the battery may be so small that it enables a run (overrun) of the blower for 5min to 15min after the power supply provided by the welding machine is removed.

In other embodiments, the battery may be sized with the object that the blower may be operated independently, without the need for the welding machine for a comparatively short period of time. This may enable the operator to move more freely, away from the welding machine 102, while still being protected by the clean air supply of the blower 130. The battery of this embodiment may be sized for independent operation of the blower, i.e. without external power supplies, for roughly 4 hours. In some examples, the battery of this embodiment may be a two-cell battery.

In the above scenario, a control unit of the blower 130 may monitor the remaining battery life and inform the user, e.g. via an audible alarm, when the remaining battery life drops below a safety threshold (e.g. five minutes of battery life remaining). In this example, the operator may use the small battery of the blower intermittently in order to access hard-to-reach places of the work piece without being limited by the blower cable 132. Yet, due to the low battery alarm of the blower, the operator can be sure that they will not be left without clean air, despite temporarily disconnecting from the power supply (i.e. the welding unit 102). Once the operator receives the safety alarm, there are two options. Firstly, the operator could leave the hazardous environment within the remaining time during which fresh air by the blower is still available. Alternatively, the operator may decide to (re-)connect the blower 130 to the welding machine 102.

In the above example, the blower 130 and the second power output 110 of the welding machine 102 are configured in such a way that, when the blower 130 is connected to the welding machine 102, the welding machine 102 not only provides sufficient power to drive the blower 130, but also to recharge the back-up battery at the same time. The blower 130 of this example could be considered as a hybrid blower that is configured to be powered by the welding machine but, at the same time, is also capable of running independently via the back-up battery for a short period of time (e.g. 4h).

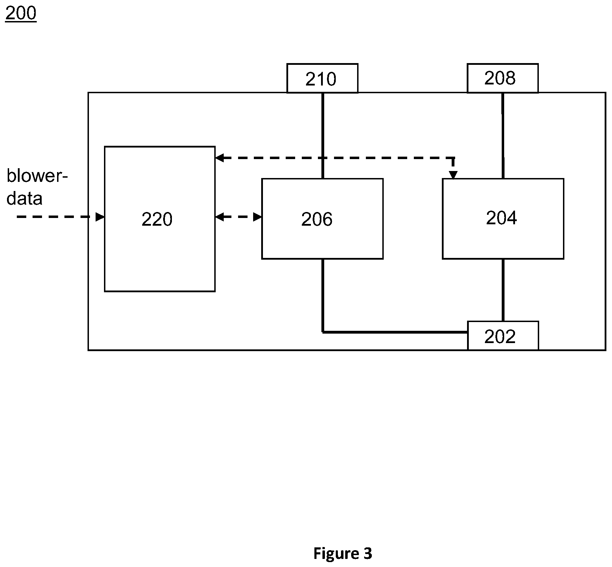

Figure 3 shows a schematic diagram of some of the internal parts of a welding machine 200 according to an embodiment of the present invention. The welding machine 200 comprises a power inlet 202 that may be connected to a mains power supply. The power inlet 202 may be provided with single or 3-phase power via the mains supply. The welding machine 202 further comprises a first converter 204 and a second converter 206. The first converter 204 is connected to the power inlet 202 and a first power outlet 208. The second converter 206 is connected to the power inlet 202 and a second power outlet 210.

Both the first and second converters 204, 206 may include various electrical components for converting the mains power 202 into the voltage and current required at the power outlets 208, 210. The relevant technical components, such as transformers and rectifiers are well-known in the art. The first convertor 204 converts the mains power supply via the power inlet 202 into a first power output that is provided to the welding tool via the first power outlet 208. The second converter 206 is configured to convert the mains power provided by the power inlet 202 into a second power output that is provided to the blower (not shown) via the second power outlet 210.

The welding machine comprises a control unit 220. The control unit 220 is in communication with the first and second convertors 204, 206. The control unit 220 may be configured to change the settings of the convertors 204, 206 either automatically or in response to a user's selection. With particular reference to the first converter 204, the control unit 220 may change the settings of the first converter 204 in response to a user's power selection via a selector (e.g. rotary dial 108 shown in Figure 2) that is arranged on the welding machine 102.

In its simplest form, the second convertor 206 may be a constant power output convertor, meaning that the second power output provided by the second convertor 206 for the blower will always remain the same and cannot be changed. In other embodiments described below, however, the second converter 206 is adjustable, e.g. via the control unit 220. This adjustable second power output enables the welding machine 200 to supply suitable power inputs to a variety of different blowers.

The control unit 220 may be configured to receive blower-data representative of one or more blower characteristics. The blower-data may be provided by the blower itself, e.g. via a control unit or communications module of the blower. In other embodiments, blower-data may be entered by the operator, e.g. via a touchscreen arranged on the welding machine 200.

In the example of Figure 2, the blower-data may be provided by the blower and transferred from the blower 130 to the relevant unit 102 via the blower cable 132 (e.g. a USB-C cable). Alternatively, it is of course also feasible to provide a separate cable for transfer of the blower-data. In yet another alternative, the blower-data may be transferred by wirelessly between the blower and the control unit 220 of the welding machine.

The control unit 220 is configured to determine, on the basis of the blower-data, a suitable input power for the blower. The control unit 220 may then adjust the second power output provided via the second power outlet 210 such that it matches the required blower input power determined above. A non-exhaustive list of possible blower-data and corresponding adjustments of the second power output are described in more detail below:
In one example, the blower-data comprises blower manufacturer details. Although blowers that may be plugged into a welding machine are not currently available, it will be appreciated that such plug-in blowers will often require a specific power input chosen by the manufacturer of the blower. In other words, most blower manufacturers will tend to operate their blowers at similar input power requirements. Accordingly, the control unit may be configured to look-up the required input power of the blower via the manufacturer details provided by as the blower-data. The name of the blower manufacturer may either be provided by the blower via a corresponding communications module or entered into the control unit 220 of the welding machine 200 directly by the user. The control unit 220 may have access to a database including manufacturer names and their corresponding blower power requirements.

In another example, the blower-data comprises blower model details. Similar to the above, the control unit 220 of this embodiment may determine the required power input of a blower on the basis of its model number provided as part of the blower-data. The model number may either be provided by the blower via a corresponding communications module or entered into the welding machine 200 by the user. If the blower model details are provided by the user, the welding machine may comprise a suitable interface, e.g. a touch screen panel.

In another embodiment, the blower-data may comprise current impeller speed. In this example, the blower-data may be provided by a speed sensor connected to the impeller of the blower or the electric motor driving the impeller. On the basis of the current impeller speed the control unit 220 may determine the need for an adjustment of the second power outlet. In particular, the control unit 220 may be able to determine a desired impeller speed, e.g. on the basis of a user input or via a look-up table, etc. Based on the current impeller speed that is part of the blower-data, the control unit 220 may determine a difference between the current impeller speed and the desired impeller speed. If a difference between the current impeller speed and the desired impeller speed exceeds an impeller speed threshold, the control unit 220 may adjust the second converter 206 in such a way that the second power outlet 210 is either increased or decreased so as to realign the current impeller speed with the desired impeller speed. If, for example, the current impeller speed is lower than the desired impeller speed, the control unit 220 may adjust the second converter 206 in such a way that the second power outlet is increased in order to increase the impeller speed. If, on the other hand, the impeller speed is too high, the control unit may adjust the second convertor 206 so as to decrease the impeller speed. The welding machine of this embodiment may thus be able to control the impeller speed continuously or intermittently via the above control unit. The control unit 220 may increase the second power output if the difference between a desired impeller speed rate and the current impeller speed falls below a lower air flow threshold. The controller may decrease the second power output if the difference the current impeller speed and the desired impeller speed exceeds an upper air flow rate threshold.

In yet another embodiment, the blower-data may comprise a current air flow rate provided by the blower. The current air flow rate may be provided by a flow rate sensor arranged between the impeller and the welding helmet. The control unit 220 may be configured to determine a desired air flow rate. The desired air flow rate may either be entered by a user via interfaces of the welding machine 200 or it may be determined by the control unit 220, e.g. on the basis of the type of a type of welding helmet used. In this regard, it will be appreciated that different types of welding helmets may have different air flow requirements, such that the control unit may be configured to adjust the second power output via the second convertor 206 to cause the impeller to provide the required air flow rate. Similar to the impeller speed, the control unit 220 may be able to adjust the current air flow rate via a control loop. In particular, the control unit 220 may increase the second power output if the difference between a desired air flow rate and the current air flow rate falls below a lower air flow threshold. The controller may decrease the second power output if the difference the current air flow rate and the desired air flow rate exceeds an upper air flow rate threshold.

The control unit 220 may be configured to receive welding-data representative of current welding parameters of the welding machine. The control unit may then determine, on the basis of the welding-data, a suitable impeller speed to produce a suitable air flow rate supplied to a respirator (i.e. the welding helmet). The control unit 220 may then provide a control-signal for adjusting the second power output provided to the blower. The welding-data may be one or more of a plurality of different data provided by one or more parts of the welding machine. A non-exhaustive list of the examples of the welding-data is shown below:
In one embodiment, the welding-data may comprise a welding current. On the basis of the welding current, the control unit 220 may increase or decrease the second power output so as to change the amount of air flow provided by the impeller of the blower. In one example, the control unit 220 may increase the second power output and thus the impeller speed as the welding current increases, due to the potential for increased production toxic fumes at higher welding currents.

In another embodiment, the welding-data may include the welding voltage. Similar to the welding current, the control unit may be configured to determine that a change in air flow rate is required if the welding voltage increases or decreases significantly.

In yet another embodiment, the welding-data may comprise a feed rate at which a wire filler metal is fed into the weld. The feed rate may be provided to a control unit 220 of the welding machine 200 by a wire fee unit. The wire feed unit may communicate with the control unit 220 of the welding machine 200 via any known data connection, such as wired or wireless connections. Accordingly, the wire feed unit may include a control unit or a communication module that is configured to communicate with the control unit 220 of the welding machine 200.

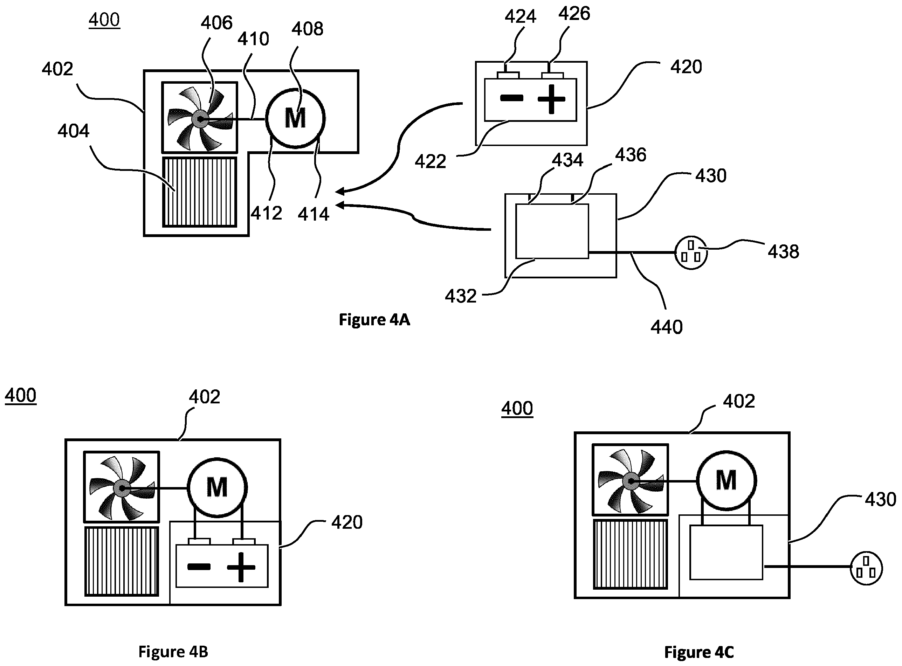

Turning to Figures 4A to 4C, there is shown schematic representations of a blower according to the present disclosure. The blower 400 shown in Figures 4A to 4C is a modular blower. Accordingly, the blower 400 comprises a modular housing. The modular housing comprises a first housing part 402. The first housing part 402 includes a filter element 404, an impeller 406 and an electric motor 408. The electric motor 408 is configured to drive the impeller 406. To this end, a drive shaft 410 of the electric motor is connected to the impeller 406. The first housing part 402 can be considered as a permanent part of the modular housing.

The modular housing of the modular blower 400 also comprises exchangeable housing parts. A first exchangeable housing part 420 is removably connectable to the first housing part 402. The first exchangeable housing part 420 comprises a battery 422 for supplying the motor 408 of the first housing part 402 with electrical power, when the first exchangeable housing part 420 is connected to the first housing part 402 (see Figure 4B).

The first exchangeable housing part 420 comprises negative and positive terminals 424, 426 adapted to align with corresponding terminals 412, 414 of the motor 408, when the first exchangeable housing part 420 is connected to the first housing part 402. In view of the above, the first configuration of the modular blower 400, shown in Figure 4B, is a configuration in which the housing comprises a removable battery 422 for supplying the blower with power. In other words, the modular blower in its first configuration shown in Figure 4B is a blower with a removable battery.

The modular blower 400 of Figures 4A to 4C also includes a second exchangeable housing part 430. The second exchangeable housing part 430 comprises a power converter 432 for converting mains power into the electrical power required by the motor 408. The converter 432 may thus comprise various known parts, such as transformers, rectifiers and smoothing capacitors, etc. to achieve the required conversion of the mains power supply to the input requirements of the motor. The convertor 432 may be specifically configured for a certain type of blower, i.e. a particular type of motor 408. In other words, the converter 432 may only convert the mains power supply into one predetermined output power suitable for the electric motor 408 of a specific modular blower 400. The converter 432 is connected to a mains power plug 438 via a mains lead 440.

The second exchangeable housing 430 may be removably connected to the first housing part 402 of the blower 400. When the second exchangeable housing part 430 is connected to the first housing part 402, terminals 434 and 436 of the converter 432 align with corresponding terminals 412, 414 of the motor 408. The modular blower 400 is then in a second configuration (Figure 4C), in which the housing comprises a removable power converter 432 for supplying the blower with power. As mentioned above, the removable power convertor 432 is connectable to a mains power outlet by the mains power plug 438. Therefore, in its second configuration, the modular blower 400 is a plug-in blower. In this second configuration shown in Figure 4C, the blower 400 is significantly lighter than the blower in its first configuration shown in Figure 4B.

In some embodiments (not shown), the second exchangeable housing part may also include a small back-up battery as a safety measure if the main power supply fails, similar to what has been described with respect to the welding kit above and shown in Figure 2.

The first and second exchangeable housing parts 420, 430 have a substantially identical shape. In other words, the size and contours of the first and second exchangeable housing parts 420, 430 are substantially the same, such that the modular housing of the blower 400 shown in Figures 4A to 4C has substantially identical aa shape/contours in its first and second configuration. This has the advantage that the modular blower 400 may be carried in the same way by the user whether the modular housing 400 is in its first configuration (Figure 4B) or in its second configuration (Figure 4C).

## Claims

1. A blower for a powered air purifying respirator (PAPR), the blower comprising a modular housing including:
- a first configuration, in which the housing comprises a removable battery for supplying the blower with power, and
- a second configuration, in which the housing comprises a removable power converter for supplying the blower with power, the removable power converter being connectable to a mains power outlet,
wherein the modular housing has substantially identical contours in its first and second configuration.

2. The blower of Claim 1,
wherein the removable power converter comprises a transformer and/or an AC/DC converter.

3. The blower of Claims 1 or 2,
wherein the removable power converter is connectable to a mains power outlet via a welding machine to receive a power input.

4. A welding machine for providing power to a welding tool, wherein the welding machine is configured to power a blower of a powered air purifying respirator.

5. The welding machine recited in Claim 4,
wherein the welding machine is connectable to a mains power supply.

6. The welding machine of Claims 4 or 5,
wherein the welding machine is configured to receive an input power and provide a first power output for powering the welding tool and a second power output for powering a blower, wherein the first power output is preferable different from the second power output.

7. The welding machine of Claim 6,
wherein the first power output and/or the second power output is different from the input power.

8. The welding machine of any one of Claims 4 to 7,
wherein the welding machine is configured to vary the first power output to vary the speed of an electric motor of the blower.

9. The welding machine of any one of Claims 4 to 8,
comprising a controller, wherein the controller is configured to:
- receive blower-data representative of one or more blower characteristics;
- determine, on the basis of the blower-data, a suitable input power for the blower; and
- adjust a second power output of the welding machine for powering the blower to said input power.

10. The welding machine of Claim 9,
wherein the blower-data comprises one or more of:
- blower manufacturer details;
- blower model details;
- a current impeller speed;
- a current air flow rate provided by the blower.

11. The welding machine of any one of Claims 4 to 10,
comprising a controller, wherein the controller is configured to:
- receive welding-data representative of current welding parameters of the welding machine;
- determine, on the basis of the welding-data, a suitable air flow rate of purified air to a respirator; and
- provide a control-signal for adjusting a second power output provided to the blower.

12. The welding machine of Claim 11,
wherein the welding-data comprises one or more of:
- a welding current;
- a welding voltage;
- a feed rate.

13. The welding machine of any one of Claims 4 to 12,
wherein the welding machine and the blower are connected via a single cable configured to transfer power and data.

14. A welding kit comprising:
- a welding machine according to any of Claims 4 to 13; and
- a blower for providing purified air to a respirator.

15. The welding kit of Claim 14,
wherein the blower comprises a battery configured to be recharged when the blower is powered by the welding machine, the battery preferably being sized to provide a maximum of 2h to 4h of blower runtime, when the blower is not powered by the welding machine, and/or
wherein the welding machine and the blower are connected or connectable via a single cable configured to transfer power and data.
